# EUROPEAN PATENT APPLICATION

(11) **EP 4 353 269 A1**
(43) Date of publication of application: **17.04.2024**
(21) Application number: 22201519.0
(22) Date of filing: 14.10.2022
(51) Int. Cl.: A61L 2/00, A61L 2/10, B65B 55/08, B65D 65/16, C08L 23/02, C08L 27/02, C08L 75/04

(54) **METHOD FOR STERILIZATION OF FORMED ARTICLES MADE OF THERMOPLASTIC POLYMERS**

(71) Applicant: Röhm GmbH, 64295 Darmstadt (DE)
(72) Inventor: BERNHARD, Dt., Kay, 64291 Darmstadt (DE); RICHTER, Dr., Ralf, 63457 Hanau (DE); HEYL, Dr., Dirk, 64295 Darmstadt (DE)
(74) Representative: Röhm Patent Association

(57) **Abstract**

The present invention relates to formed articles, such as medical devices, made of an thermoplastic polymer material, which comprises at least one of thermoplastic polymer A, selected from cyclic polyolefins; aliphatic polyamides, aliphatic polyurethanes and halogenated polyolefins, and a method for their UV disinfection and/or sterilization, including disinfection and/or sterilization of the inner surface of the formed article. The inventive method for UV sterilization comprises the exposure of the outer surface of the formed article with UV radiation at a wavelength in the range of 260 nm to 300 nm, wherein the thermoplastic polymer material has a transmittance of at least 10 %, averaged over the wavelength range from 260 nm to 300 nm, measured in accordance with ISO 13468-2 at a thickness of 3 mm.

## Description

### Technical field

The present invention relates to a method for UV disinfection and/or sterilization of formed articles, such as medical devices, made of thermoplastic polymer material, which comprises at least one thermoplastic polymer A selected from cyclic polyolefins, aliphatic polyamides, aliphatic thermoplastic polyurethanes and halogenated polyolefins, including disinfection and/or sterilization of the inner surface of the formed article. The inventive method for UV sterilization comprises the exposure of the outer surface of the formed article with UV radiation at a wavelength in the range of 260 nm to 300 nm, wherein the thermoplastic polymer material has a transmittance of at least 10 %, averaged over the wavelength range from 260 nm to 300 nm, and measured in accordance with ISO 13468-2 at a thickness of 3 mm.

### Background art

Medical grade thermoplastic polymer materials, such as cyclic polyolefins, thermoplastic polyurethanes (TPU), polyamides (PA), and polymethylmethacrylate (PMMA), are commonly known in the state of the art and typically offer a desired balance of optical and mechanical properties. These polymer materials are often transparent, have a good thermoplastic processability and can be advantageously used for production of medical devices, e.g. via injection molding or extrusion. Typical applications of such devices include commonly known medical and medical diagnostic applications, such as intravenous and catheter accessories, blood handling devices, chest drainage units, and respiratory ventilating devices.

Generally, medical devices, especially disposables devices intended for intravenous application (e.g. IV-Sets), need to be sterilized before use. This is usually done by the manufacturer, so the devices are ready to use. Medical devices made from known thermoplastic polymer materials are often sterilized using chemical sterilization agents, such as ethylene oxide, or using gamma rays, e-beam or X-radiation. As a rule, only a small share of about 10% is sterilized by X-ray or electron beam exposure, most of such medical devices are sterilized by gamma radiation or ethylene oxide treatment (each about 45%). Both procedures are well established in the medical industry and considered state of the art, but they involve a lot of disadvantages.

In the case of sterilization using gamma radiation, radioactive radiation and substances, mainly Co-60 (t_{1/2} = 5.27 a) need to be handled. That requires a lot of investment and very strict processes. Facilities for gamma radiation sterilization need to be rather large to compensate for these investments via economy of scales. Lately a shortage of Co-60 has been reported. In the case of sterilization using ethylene oxide, a similar level of precautions is needed, because ethylene oxide is extremely flammable and carcinogenic. This process also needs certain requirements in packaging since the ethylene oxide needs to reach the medical devices directly. Facilities also need to be rather large to compensate for these investments via economy of scales. Sometimes ethylene oxide sterilization sites have to be closed because of too high ethylene oxide exposure.

The sterilization effect of ultraviolet (UV) irradiation has been known since the latter half of the 19th century, and in recent years, the use of UV irradiation has been widely accepted in the field of water and air purification, as well as in disinfection and sterilization in food processing or of medical equipment. For example, UV radiation is used for disinfection in hospitals, nurseries, operating rooms, cafeterias and to sterilize vaccines, serums, toxins, municipal waste, and drinking waters. Generally, UV light is separated into the categories of UV-A (typically of wavelength range from 315 to 400 nm), UV-B (typically of wavelength range from 280 to 315 nm), and UV-C (typically of wavelength range from 200 to 280 nm).

Specifically, UV radiation does not kill microorganisms directly, but damages the genetic material, the deoxyribonucleic acid (DNA), of the microorganisms, e.g. pathogens. This inhibits the cell division ability and effectively kills the microorganism. Generally, UV-C radiation have been shown to initiate a photoreaction between adjacent pyrimidine bases, such as cytosine and thymine, which exhibit conjugated double bonds and as such absorb UV light. The germicidal effectiveness of UV-C radiation peaks at about 260-265 nm and typically corresponds to the maximum UV absorption by bacterial DNA. In particular, the optimum wavelength for germicidal effectiveness varies between different microorganism species. Generally, UV radiation in UV-B range only have a small contribution to the inactivation of microorganisms (W. J. Kowalski, "UVGI Disinfection Theory", in Ultraviolet Germicidal Irradiation Handbook, pp. 17-25, July 2009, DOI: 10.1007/978-3-642-01999-9_2).

Generally, the photoreaction between adjacent thymine or cytosine bases proceeds at an exceedingly rapid rate, wherein one common reaction is the formation of a cyclo-butane ring between the two pyrimidines. The inactivation of specific genes via punctual mutations is one of the mechanisms of UV-induced genetic damage that effect inhibition of cell replication and cell death (W. J. Kowalski, "UVGI Disinfection Theory", in Ultraviolet Germicidal Irradiation Handbook, pp. 17-25, July 2009, DOI: 10.1007/978-3-642-01999-9_2).

UV-C radiation in the range of 220-280 nm is widely used in industry for disinfection of liquids, e.g. water, and surfaces. Since water is UV transparent at 220 nm, this is a very common wavelength for disinfection of well water. Mercury UV lamps, which are widely used for disinfection and sterilization, emit at 254 nm. Further, DNA specifically has a high absorbency at said wavelength. Thus, wavelength of 254 nm has been and still is widely used in prior art germicidal devices. Indeed, with the development of UV-LEDs the range of 260-280 nm became accessible.

It is well-known that most of common transparent polymeric materials, such as polycarbonates (PC), polyethylene terephthalates (PET), have only a limited transmittance of UV light, in particular have a low or no transmittance below 280 nm. Therefore, often complex parts cannot be UV sterilized since the UV radiation does not reach inner surfaces, voids, and cavities. Polymers containing aromatic moieties like polystyrene and polyethylene terephthalates, are intrinsically not UV transparent. Other thermoplastic polymers, like PVC, PMMA, cyclic polyolefins, polyurethanes and polyamides, often comprises at least some UV absorbing additives, such as softeners (phthalates) or stabilizer. Pure cyclic polyolefins, such as cyclic olefin polymers COP, cyclic olefin copolymers COC, and cyclic block copolymers CBC; aliphatic polyamides, aliphatic polyurethanes and halogenated polyolefins, often show a good transmittance in the UV-A range (320-400 nm) and some transmittance in UV-B and UV-C. In practice, however, certain processing additives, such as UV stabilizers, may lead to reduced UV transmittance of said thermoplastic materials.

The document US 7,834,328 B2 describes a method and apparatus for sterilizing access sites, such as attachment points for various therapeutic and diagnostic medical devices. More particularly, US 7,834,328 B2 concerns a sterilization apparatus which includes a substantially UV-C transparent closure cap for closing the access site and an irradiating apparatus for irradiating the closure cap with UV-C radiation. Typically, said closure can be built up entirely from UV-C transparent materials or comprises a hollow, substantially UV-C opaque body; and a substantially transparent part. The UV transparent material is for example selected from quartz glass or polyethylene. Sterilizing is carried out using UV-C radiation in the wavelength range of 200 to 280 nm.

The document JP5938408B2 describes a vascular access device including a fluid chamber having a lumen, an inner surface, an outer surface, and an irradiation window, wherein the irradiation window is made of a UV-C transmissive material so that pathogens in the lumen can be irradiated with UV-C radiation of 250 to 265 nm. For example, the illumination window is made of optical quartz or fluorinated polymer material.

EP 1 033 326 A1 describes a container for medical device, e.g. a container for contact lens, wherein said container is transmissive over substantially all of the surface area to radiation in the range of 240 to 280 nm. The container comprises a multilayer material including, for example, cyclic olefine copolymer, polyvinylchloride, polyflurocarbon, polyurethane, polyamide or polymethylmethacrylate.

WO 2019/237001 describes devices, such as door opening means and switches, comprising a UV-transparent or translucent article and an optically connected UV-light source, wherein the surface of the device should be automatically disinfected using internally generated UV light. Suitable UV-transparent or translucent materials used for said article may be acrylic glass, polyethyleneterephthalate PET, cyclic olefin copolymer COC and glass, quartz glass. The UV light source should emits light in wavelength range from 100 to 400 nm, in particular in the UC-C range, preferably 250 to 260 nm, e.g. 254 nm.

Further, US 2013/0078142 A1 describes a method of UV-C radiation sterilization, wherein the relevant object is placed into a contained and sealed. At least a portion of this container is substantially transparent to UV-C radiation in the range of 235 to 295 nm and the container is irradiated with UV-C radiation in the relevant range. Suitable UV transparent materials are quartz glass, borosilicate glass, cyclic olefin copolymer, and fluoropolymer.

Several known medical grade polymer compositions comprise copolymers of including aromatic vinyl monomers, often particulate impact modifier and/or further polymeric component (e.g. WO 2020/126722 A1, WO 2008/148595 A1, and JP H02-272050 A2).

Such medical grade polymer compositions already have a good chemical resistance to alcohols, which are commonly used as disinfectants for medical devices, but they tend to become turbid and get cracks upon a long-term exposure to isopropanol-water mixtures. Further, such known medical grade polymer compositions are not suitable for UV sterilization, in particular not for UV sterilization of their inner surface, due to their low transmittance for UV-C radiation.

Thus, there is the need for novel, easy and effective methods for sterilization of medical devices, in particular disposable medical devices, avoiding the impairment of optical and mechanical properties of said medical devices.

An object of the invention is to provide formed articles, such as medical devices, which exhibit excellent mechanical and optical properties, e.g. high transparency, and which can be easily and effectively be sterilized using UV radiation, including the inner surface of the formed article. Another object is directed to the corresponding easy and cost-effective method for UV sterilization of the inner surface of said formed articles. Further, an object of the present invention is to provide formed articles, such as disposable medical devices, which can be produced easily and cost-effectively via a thermal molding process, e.g. via injection molding.

### Disclosure of the invention

Generally, pure thermoplastic polymers, selected from cyclic polyolefins (such as COP, COC, and CBC); aliphatic polyamides, aliphatic polyurethanes and halogenated polyolefins which are completely or nearly free of UV absorbing additives, provide a certain transparency to UV-C light at 265 nm. Typically, up to 25 % of such UV-C light pass a pure thermoplastic material having 3 mm thickness and stay effective towards germs. Radiation of higher wavelength up to 280 nm in UV-B and UV-A ranges, is less effective, but the transmittance of said thermoplastic is often higher in said range. Therefore, this ranges of transmitting UV radiation, preferably from 280 nm to 300 nm, can be used for sterilization and/or disinfection as well.

It was surprisingly found that the UV transmittance of specific thermoplastic materials is high enough to provide an effective and safe sterilization and/or disinfection of the inner surface of arbitrary formed articles made of said thermoplastic material, when UV radiation in the range of 260 to 300 nm is used. Further, said thermoplastic material can be a combination of two or more of said thermoplastic polymers or combination of one or more of said thermoplastic polymers with other UV-C transparent polymers, like polymethylmethacrylate (PMMA), or silicone.

Particularly, said thermoplastic polymers, selected from cyclic polyolefins (such as COP, COC, and CBC); aliphatic polyamides, aliphatic polyurethanes and halogenated polyolefins, in combination with certain additives and/or stabilizers, exhibit a good transmittance in the relevant UV range, i.e. at least 10 %, averaged or summarized over the range of 260 to 300 nm, preferably of 260 to 280 nm, most preferably at 265 nm. Furthermore, other important optical and mechanical properties of said thermoplastic polymers and formed articles made thereof are good to excellent as well.

Therefore, these said thermoplastic polymers, selected from cyclic polyolefins (such as COP, COC, and CBC); aliphatic polyamides, aliphatic polyurethanes and halogenated polyolefins, can be advantageously used as material for formed articles, that are subject to a UV sterilization process, such as medical devices, e.g. disposable medical devices, or various containers and/or packaging used in food industry, pharmaceutical industry or cosmetic industry.

Advantageously, it is possible to achieve a sterilization and/or disinfection of the whole surface of the formed article, including the inner surface of the formed article, for example the inner surface of openings and/or cavities of the formed article.

The present invention is directed to a method for UV sterilization of a surface of a formed article, such as a medical device or a food container, including sterilization of the inner surface of the formed article, comprising the steps:
a. providing a formed article, wherein at least one part of said formed article is made from (consists of) a thermoplastic polymer material, which comprises at least 50 wt.-%, preferably at least 60 wt.-%, more preferably at least 70 wt.-%, based on the thermoplastic polymer material, of at least one thermoplastic polymer A, selected from cyclic polyolefins, such as cyclic olefin polymers COP, cyclic olefin copolymers COC and cyclic block copolymers CBC; aliphatic polyamides, aliphatic polyurethanes and halogenated polyolefins;
b. exposure of the outer surface of the formed article with UV radiation at a wavelength in the range of 260 to 300 nm, preferably 260 nm to 280 nm;
   wherein the thermoplastic polymer material has a transmittance of at least 10 %, preferably at least 15 %, more preferably at least 20 %, also preferably at least 25 %, averaged over the wavelength range from 260 nm to 300 nm, preferably from 260 to 280 nm, and measured in accordance with ISO 13468-2 at a thickness of 3 mm.

A further aspect of the present invention relates to a formed article, in particular for use as medical device, in food, cosmetic and/or pharmaceutical industry, comprising at least one part made of the thermoplastic polymer material. Importantly, the inventive formed articles exhibit an advantageous balance of properties, including high transmittance in the relevant UV range needed for sterilization as well as a number of further advantageous properties, such as excellent biocompatibility, low haze (i.e. high transparency in the VIS range), excellent processability, good mechanical properties, such as good scratch resistance, high heat distortion resistance, high modulus of elasticity and/or high Vicat softening temperature.

The thermoplastic polymer material can be prepared and processed in a relatively simple manner and is particularly suitable for the production of articles having a complex geometrical shape, using a commonly known thermal molding process, in particular injection molding. Hence, in this further aspect, the present invention relates to a process for producing the formed article from the thermoplastic polymer material, preferably via injection molding.

Finally, a further aspect of the present invention is directed to the use of the inventive formed article in a method for UV sterilization, wherein UV radiation at a wavelength in the range of 260 nm to 300 nm, preferably 260 nm to 280 nm, is utilized.

### Detailed description of the invention

Often sterilization is understood as a process of complete elimination or destruction of all forms of microorganisms, including both vegetative and spore forms, preferably from inanimate objects, wherein sterilization may be carried out by various physical and chemical methods. Often sterilization is achieved by dry or moist heat, irradiation or gassing with ethylene oxide, formaldehyde or vaporized hydrogen peroxide (VHP). Often disinfection is understood as a process of reduction of the number of microorganisms by killing or irreversibly inactivating vegetative forms of microorganisms, e.g. pathogenic microorganisms, except bacterial spores, from inanimate objects and to interrupt infection chains. Often decontamination is defined as a process of removal of pathogenic microorganisms from inanimate objects, so that they are safe to handle (S. Mohapatra, Sterilization and Disinfection, Essentials of Neuroanesthesia. 2017 : 929-944, Clinical and Laboratory Standards Institute).

In terms of the present invention the term *"sterilization"* or *"method for sterilization*" includes sterilization, disinfection and/or decontamination. In particular, the term *"sterilization"* or *"method for sterilization"* according to the invention encompasses the reduction of the number of microorganisms by killing and/or irreversibly inactivating growth of microorganisms. Preferably, the inventive process results in a colony forming units (CFU) log reduction on the inner surface of the formed article of at least 1, more preferably at least 2, more preferably at least 3, more preferably at least 4, also preferably at least 5, even more preferably of at least 6, of relevant microorganisms, preferably of vegetative forms of microorganisms.

In terms of the present invention *"inner surface of the formed article"* means the surface of the formed article, which is not directly exposed to the UV radiation and/or which is opposite to the surface exposed to the UV radiation. Typically, the inner surface may include openings, cavities and/or pores, which can typically not or only with high difficulties be exposed to UV radiation directly.

As used herein, the term *"ultraviolet radiation"* or *"UV radiation"* refers to radiation having a wavelength or wavelengths in the range from 200 to 400 nanometers (nm). If a range is specified, such as 260 nm to 300 nm, the range specified, unless otherwise indicated, means radiation including emission at one or more wavelengths within this specified range.

As used herein, the abbreviation "*UV-A*" refers to ultraviolet radiation in the range of ≥ 315 nm to ≤ 400 nm. As used herein, the abbreviation *"UV-B"* refers to ultraviolet radiation in the range of > 280 nm to < 315 nm. As used herein, the abbreviation *"UV-C"* refers to ultraviolet radiation in the range of ≥ 200 to ≤ 280 nm.

As used herein, the term *"ultraviolet and visible radiation"* or "*UV*/*Vis radiation"* refers to radiation having a wavelength or wavelengths in the range from 200 nm to 800 nm, preferably 240 to 780 nm.

In terms of the present invention the term *"polymer or copolymer comprising or consisting of monomer(s)"* is understood in that the polymer or copolymer comprises or consists of said monomer unit(s). As a skilled person understands said polymers or copolymers are obtained by polymerisation, polycondensation or polyaddition of the referred monomers. For example, at least one of the unsaturated groups of the monomers is polymerized, preferably radically polymerized. As a skilled person understands such polymer or copolymer may also include groups resulting from initiators, e.g. radical initiators and/or molecular weight regulators. In case, that the polymer obtained after said polymerization comprises unreacted monomers, which are not incorporated in the polymer chain, this is referred to as residual monomer(s).

The thermoplastic polymer material comprises (also referred to as polymer material in the following) at least 50 wt.-%, preferably at least 60 wt.-%, more preferably at least 70 wt.-%, also preferably at least 80 wt.-%, based on the total polymer material, of at least one thermoplastic polymer A, selected from cyclic polyolefins, such as cyclic olefin polymer COP, cyclic olefin copolymer COC and cyclic block copolymer CBC; aliphatic polyamides, aliphatic polyurethanes and halogenated polyolefins.

According to a preferred embodiment the thermoplastic polymer material comprises from 50.0 to 100.0 wt.-%, preferably 60.0 to 99.9 wt.-%, more preferably 70.0 to 95.0 wt.-%, based on the total polymer material, of at least one thermoplastic polymer A selected from cyclic olefin polymer COP, cyclic olefin copolymer COC, cyclic block copolymer CBC; aliphatic polyurethanes and aliphatic polyamides, and halogenated polyolefins, preferably homo- or copolymer comprising at least one di- or tri- halogenated olefinic monomer.

Preferably, the thermoplastic polymer A is selected from transparent polymers. More preferably the thermoplastic polymer material may be a blend, in particular a transparent blend, of two or more thermoplastic polymers A, wherein the thermoplastic polymers A are compatible and miscible with each other. In terms of the present invention, the term "transparent" refers to polymers or polymer compositions having a light transmission of at least 50 %, measured according to ASTM D1003, using an Illuminate C light source.

### Cyclic polyolefins

Preferably the thermoplastic polymer A comprises or consists of at least one cyclic polyolefin, wherein cyclic polyolefins are typically polymers having a saturated hydrocarbon cyclic structure in the main chain or side chain of polymers. Preferably, cyclic polyolefins used as thermoplastic polymer material are selected from cyclic olefin polymers COP, cyclic olefin copolymers COC, and cyclic block copolymers CBC as described below.

Preferably, the cyclic olefins in the thermoplastic polymer material are non-crystalline and transparent resins.

According to a preferred embodiment, the thermoplastic polymer material comprises from 50.0 to 100.0 wt.-%, preferably 60.0 to 99.9 wt.-%, more preferably 70.0 to 95.0 wt.-%, based on the total polymer material, of a thermoplastic polymer A selected from cyclic polyolefins, which may preferably be cyclic olefin polymers COP, cyclic olefin copolymers COC, and cyclic block copolymers CBC.

### Cyclic olefin polymers (COP)

Preferably the thermoplastic polymer A comprises or consists of at least one cyclic olefin polymers COP. Typically, COP is a polymer of at least one cyclic olefinic monomer, preferably including norbornene, produced via ring opening metathesis polymerization (ROMP), typically using a metatheses catalyst, such as ruthenium or osmium metal carbene metathesis catalyst.

Preferably, the thermoplastic polymer material comprises from 50.0 to 100.0 wt.-%, preferably 60.0 to 99.9 wt.-%, more preferably 70.0 to 95.0 wt.-%, based on the total polymer material, of a thermoplastic polymer A selected from one or more cyclic olefin polymers COP.

Typically, COP are produced via ROMP using a single type of cyclic olefinic monomer, preferably norbornene. Said cyclic olefin polymers obtained by ROMP may be hydrogenated at least partially after polymerization. For example, suitable COP are disclosed in WO 99/02585 A1 and US-B 6,682,797 B1.

Typically, the at least one cyclic olefinic monomer is selected from cyclic and multicyclic olefins containing between 6 and 200 carbon atoms, preferably selected from, dicyclopentadiene (DCPD), cyclopentadiene oligomers including trimers, tetramers, pentamers, and the like (preferably tricyclopentadiene, tetracyclododecene) substituted dicyclopentadienes, including for example alkyl substituted dicyclopentadienes; cyclobutene, cycloheptene, cyclooctene, cyclononene, cyclodecene, cyclooctadiene; cyclononadiene, cyclododecatriene, norbornene; and substituted norbornes including for example alkyl substituted norbornene, such as butyl norbornene, hexyl norbornene, octyl norbornene, decyl norbornene, or combinations thereof.

For example, suitable cyclic olefin polymers (COP) have a melt flow (measured according to ASTM D1238 (21.8 N kg load and 280 °C) in the range of 6 to 20 cm³/10 min and a glass transition temperature in the range of 70 to 160 °C.

Preferably, the thermoplastic polymer A may be selected from transparent cyclic olefin polymers (COP), for example commercially available products, such as ZEONEX^{®} and ZEONOR^{®} products from ZEON Speciality Materials, Inc, in particular ZEONEX^{®} and ZEONOR^{®} medical grades, such as ZEONOR^{®} 1020R, ZEONEX^{®} 5000, ZEONEX^{®} 690R, and ZEONEX^{®} 790R. Further commercially available COP products are APEL^{™} from from Mitsui Chemicals.

### Cyclic olefin copolymers (COC)

Preferably the thermoplastic polymer A comprises or consists of at least one cyclic olefin copolymer COC. Typically, cyclic olefin copolymers (COC) are copolymers of at least one cyclic olefinic monomer, such as norbornene and/or tetracyclododecene, and at least one linear olefinic monomer, preferably ethylene, which are typically produced by chain copolymerization using homogeneous metallocene catalyst by an addition polymerization mechanism without ring opening.

Preferably, the thermoplastic polymer material comprises from 50.0 to 100.0 wt.-%, preferably 60.0 to 99.9 wt.-%, more preferably 70.0 to 95.0 wt.-%, based on the total polymer material, of a thermoplastic polymer A selected from one or more cyclic olefin copolymers COC.

For example, suitable COC used as thermoplastic polymer A is described in JP 2 600 295 B2; EP 0 335 985, US 6,630,234

Typically, the COC glass transition temperature (T_{g}) can be varied from about 20 to 260°C by varying the cyclic olefin comonomer content. For example, suitable cyclic olefin copolymers (COC) have a volume melt flow (measured according to ISO 1133 (2.16 kg load and 260 °C) in the range of 4 to 50 cm³/10 min.

Preferably, the suitable cyclic olefin copolymers COC used as thermoplastic polymer A comprises at least 55 wt.-%, preferably at least 60 wt.-%, more preferably from 55 to 90 wt.-%, of at least one cyclic olefinic monomer, typically norbornene.

Preferably, the thermoplastic polymer A may be selected from transparent cyclic olefin copolymers (COC), for example commercially available products, such as TOPAS^{®} COC products from TOPAS Advanced Polymers GmbH.

### Cyclic block copolymers (CBC)

Preferably the thermoplastic polymer A comprises or consists of at least one cyclic block copolymer (CBC), which is a fully hydrogenated block copolymers based on at least one vinyl aromatic monomer, such as styrene, and at least conjugated diene monomer, such as butadiene, wherein the block copolymer is typically produced via anionic polymerisation. Typically, CBC are produced via anionic block copolymerization of styrene and at least one conjugated diene, e.g. butadiene, and subsequent catalytic hydrogenation.

"Substantially fully hydrogenated" means that at least 95 %, preferably at least 99 %, of vinyl aromatic double bonds and/or at least 95 %, preferably at least 99 %, of conjugated diene double bonds are hydrogenated.

Preferably, the cyclic block copolymers (CBC) are fully hydrogenated styrene-butadiene block copolymers. Typically, the properties, such as elasticity, are adjusted via the ratio of styrene and butadiene.

The cyclic block copolymers (CBC) may be linear block copolymers, preferably having alternating blocks, such as tri-block or penta-block; multi-armed or coupled block copolymers. Typically, multi- armed and coupled block copolymers contain a residue from a coupling agent X and may be represented as X(BS)n where n is > 1.

Preferably, the CBC is selected from fully hydrogenated copolymers based on at least one vinyl aromatic monomer, such as styrene and/or alpha-methyl-styrene, and at least conjugated diene monomer, such as butadiene and/or isoprene, wherein the block copolymers are built up, prior to hydrogenation, of 1 to 20, preferably 2 to 10 alternating blocks of aromatic vinyl monomers, such as styrene (S) blocks, and blocks of conjugated diene monomers, such as butadiene (B) blocks or isoprene (I) blocks. The vinyl aromatic blocks, e.g. styrene (S) blocks, may have equal or different length. Similarly, the conjugated diene blocks, e.g. butadiene (B) blocks, may have equal or different length. but need not be of equal length.

More preferably, the CBC comprises or consists of at least one hydrogenated penta-block copolymer SBSBS of styrene and butadiene.

For example, suitable cyclic block copolymers (CBC) have a melt flow (measured according to ASTM D1238 (2.16 kg load and 260 °C) in the range of 3 to 20 cm³/10 min and a glass transition temperature in the range of 115 to 130 °C.

Preferably, the thermoplastic polymer A may be selected from transparent cyclic block copolymers (CBC), for example commercially available products, such as ViviOn^{™} (from USI Corp. Taiwan), in particular medical grades of ViviOn^{™}.

### Aliphatic polyamides (PA)

Preferably the thermoplastic polymer A comprises or consists of at least one aliphatic polyamide (PA). In general, polyamides refer to polymers in which amide linkages - C(=O)NH- occur along the molecular chain. The term aliphatic polyamides include homopolymers and copolymers, such as polyether-amide, polyester-amide, and polyether-ester-amide block copolymers. For example, amorphous transparent or translucent polyamides that may be formed from the condensation of diamines with dicarboxylic acids or lactams; are described in US WO 2009/014849. For example, polyamide copolymers, such as a copolymers containing polyether blocks and polyamide blocks, are described in US 2013/0202831.

Preferably, the thermoplastic polymer material comprises from 50.0 to 100.0 wt.-%, preferably 60.0 to 99.9 wt.-%, more preferably 70.0 to 95.0 wt.-%, based on the total polymer material, of a thermoplastic polymer A selected from aliphatic polyamides.

Preferably, the aliphatic polyamide used as thermoplastic polymer A is selected from transparent polyamides, typically having a microcrystalline structure or amorphous structure.

Typically, aliphatic polyamides for use in this invention may be obtained, via the following methods:
i) polycondensation of at least one aliphatic dicarboxylic acid, such as oxalic acid, adipic acid, sebacic acid, or 1,4-cyclohexanedicarboxylic acid, with at least one aliphatic diamine, such as ethylenediamine, tetramethylenediamine, pentamethylenediamine, hexamethylenediamine, decamethylenediamine, and 1,4-cyclohexyldiamine;
ii) ring-opening polymerization of cyclic lactam, such as ε-caprolactam or ω-laurolactam, wherein ring opening is often be carried out by anionic initiators; or
iii) polycondensation of at least one aliphatic aminocarboxylic acid, such as 6-aminocaproic acid, 9-aminononanoic acid, 11-aminoundecanoic acid or 12-aminododecanoic acid.

Aliphatic polyamides produced according to option i) are commonly designated as nylon 4.6, nylon 6.6, nylon 6.9, nylon 6.10, nylon 6.12; and the like, where the first number indicates the number of carbon atoms connecting the two amine groups in the diamine and the second number indicates the number of carbon atoms connecting the two carboxylic acid groups in the dicarboxylic acid, including those in the acid groups. For example, nylon 6.6 is the reaction product of hexamethylenediamine and adipic acid.

Aliphatic polyamides produced according to options ii) or iii) are commonly designated as nylon 4, nylon 6, nylon 7, nylon 11, nylon 12, nylon 13 and the like, where the number refers to the number of carbon atoms making up the ring in the cyclic lactam monomer or the number of carbon atoms connecting the amine group and the carboxylic acid group in the aliphatic aminocarboxylic acid . For example, nylon 6 can be obtained by ring-opening polymerization of caprolactam and nylon 12 can be obtained by ring-opening polymerization of laurolactam or by polycondensation of 12-aminododecanoic acid (ω-aminolauric acid). For example, nylon 11 is based on 11-aminoundecanoic acid and can be obtained by polycondensation.

According to a preferred embodiment the thermoplastic polymer A comprises or consists of at least one aliphatic polyamide selected from nylon 4, nylon 6, nylon 7, nylon 11, nylon 12, nylon 13, nylon 4.6, nylon 6.6, nylon 6/9, nylon 6/10, nylon 6/12, nylon 12/12, nylon 13/13, and mixtures thereof. More preferably, the at least one aliphatic polyamide is selected from nylon 6, nylon 11, nylon 12, nylon 4/6, nylon 6/6, nylon 6/9, nylon 6/10, nylon 6/12, nylon 6/66, and nylon 6/69 and mixtures thereof.

Specific examples of suitable aliphatic polyamides include nylon 6, nylon 6/6; nylon 6/10, nylon 11, and nylon 12. More preferred suitable aliphatic polyamides used as thermoplastic polymer A include nylon 11 and nylon 12.

Furthermore, it is possible to use a blend/mixture of at least one aliphatic polyamide as thermoplastic polymer A, such as nylon 6, nylon 6/6; nylon 6/10, nylon 11, and nylon 12, and at least one polyolefin, such as polyethylene and polypropylene, as polymeric component C.

For example, the thermoplastic polymer A may comprise or consists of one or more aliphatic polyamide, for example selected from commercially available transparent polyamide products, such as Grilamide TR^{®} from EMS-Chemie AG (e.g. Grilamid^{®} TR 90, Grilamid^{®} TR 30, Grilamid^{®} TR55) and EMS-GRIVORY (e.g. Grivory^{®} G and GTR), both from EMS-Chemie, products of TROGAMIDE^{®} type from Evonik (e.g. TROGAMIDE^{®} CX 7323, TROGAMIDE^{®} T5000, TROGAMIDE^{®} BX), and VESTAMID^{®} product types from Evonik. Other suitable examples include Ultramid^{®} polyamides, available from BASF; and Zytel^{™} and Dartek^{™} nylon resins, available from DuPont, and Durethan^{®} grades available from Lanxess AG (Cologne, Germany).

Transparent polyamide copolymers that may be used as thermoplastic polymer A are polyether-amide, polyester-amide, polyether-ester-amide block copolymers, such as Pebax^{®} product family from Arkema, Inc..

### Aliphatic polyurethanes (PU)

Preferably the thermoplastic polymer A comprises or consists of at least one aliphatic polyurethane (PU), in particular aliphatic thermoplastic polyurethanes (TPU). In general, polyurethane refer to polymers in which carbamate (-NH-C(=O)-O-R) linkages occur along the molecular chain. The term aliphatic polyurethane and aliphatic thermoplastic polyurethane include homopolymers and copolymers, such as polyether-urethan, polyester-urethan block copolymers. For example, TPU are described in WO 2016/142513 A1 and WO 2016/018749 A1.

Preferably, the thermoplastic polymer material comprises from 50.0 to 100.0 wt.-%, preferably 60.0 to 99.9 wt.-%, more preferably 70.0 to 95.0 wt.-%, based on the total polymer material, of a thermoplastic polymer A selected from aliphatic polyurethanes (PU), preferably from aliphatic thermoplastic polyurethanes (TPU).

Preferably, the aliphatic polyurethane is selected from transparent aliphatic thermoplastic polyurethanes (TPU), typically having a microcrystalline structure or amorphous structure.

Generally, aliphatic polyurethanes are e generally prepared by reacting at least one aliphatic multifunctional isocyanate (i.e. polyisocyanate) with at least one aliphatic multifunctional diol (i.e. polyol) in the presence of a catalyst in a polycondensation reaction. Particularly preferred aliphatic polyisocyanates include dicyclohexylmethane diisocyanate, isophorone diisocyanate, hexamethylene diisocyanate, trimethylhexamethylene diisocyanate, 4,4'-diisocyanato dicyclohexylmethane (hydrogenated MDI) along with copolymers and mixtures thereof.

Suitable aliphatic polyols include, for example, low molecular weight polyols, such as ethylene glycol, propylene glycol, 1,4-butanediol, 1,5-pentanediol, 1,6-hexanediol, 1,7-heptanediol, 1,8-octanediol and the higher homologues or isomers thereof. The term "low molecular weight diol" means a diol having a weight average molecular weight of at most 185 g/mol.

Preferred aliphatic polyols are reaction products of low molecular weight polyols, e.g. mentioned above, with alkylene oxides, preferably having 2 to 4 C atoms. Further suitable polyols are obtained by polymerization of tetrahydrofuran (poly-THF). Particularly preferred are polypropylene glycol homopolymers.

Further preferred aliphatic polyols are polyester polyols. Examples of these are polyester polyols, which are obtained by reacting low molecular weight aliphatic alcohols, in particular ethylene glycol, diethylene glycol, neopentyl glycol, hexanediol, butanediol, propylene glycol, glycerol or trimethylolpropane with caprolactone or with an aliphatic polycarboxylic acid via polycondensation.

Commonly, thermoplastic polyurethanes (TPU) are characterized by linear polymeric chains having self-ordering block structures, wherein these polyurethanes are generally uncrosslinked or lightly crosslinked. Often TPU are block copolymer consisting of alternating sequences of hard and soft segments or domains formed by the reaction of (1) diisocyanates with low molecular weight diols (so-called chain extenders resulting in hard segments) and (2) diisocyanates with long-chain diols (resulting in soft segments). The soft segments may typically be selected from polyester-polyol segments, mainly derived from adipic acid esters, resulting in polyester-based TPUs or from polyether-polyol segments, mainly based on tetrahydrofuran ethers, resulting in polyether-based TPUs.

The block structures of a thermoplastic polyurethane generally include alternating "hard" and "soft" segments covalently bonded to each other end-to-end. The hard segments aggregate to form crystalline regions that act like physical crosslinks at ambient temperatures but convert to a molten state upon heating. As a result, thermoplastic polyurethanes are well suited for thermoforming onto three dimensional articles and can be easily reprocessed.

Preferably, the thermoplastic polymer A may comprise or consists of one or more aliphatic polyether-based thermoplastic polyurethanes (TPUs) and/or one or more aliphatic polyester-based thermoplastic polyurethanes (TPUs).

For example, the thermoplastic polymer A may comprise or consists of one or more aliphatic polyether-based thermoplastic polyurethanes (TPUs), in particular transparent aliphatic polyether-based TPUs, e.g. commercially available as Tecoflex^{™} TPU, preferably Tecoflex TPU Clear types, Tecophilic^{™} TPU, or Tecobax^{™} TPU, all from Lubrizol Corporation, US.

Further aliphatic thermoplastic polyurethanes (TPUs) may be obtained as Texin^{®}, in particular Texin^{®} Rx types, from Covestro LLC, US, as well as aliphatic polyester-grades or polyether-grades of Elastollan^{®} from BASF SE.

### Halogenated polyolefins

Preferably the thermoplastic polymer A comprises or consists of at least one thermoplastic halogenated polyolefin, which is a homo- or copolymer encompassing at least one halogenated olefinic monomer, preferably a di- or tri- halogenated olefinic monomer, wherein the halogenic group is preferably selected from chloro (CI) and/or fluoro (F), such as 1-Chlor-1,2,2-trifluorethen CCIF=CF₂ or vinylidene fluoride CH₂=CF₂ (1,1 -difluoro ethylene).

Preferably, the thermoplastic polymer material comprises from 50.0 to 100.0 wt.-%, preferably 60.0 to 99.9 wt.-%, more preferably 70.0 to 95.0 wt.-%, based on the total polymer material, of a thermoplastic polymer A selected from halogenated polyolefins.

For example, commonly known halogenated polyolefins are polyvinylchloride (PVC) produced by polymerization of vinylchloride CH₂=CHCl as monomer; polyvinylidenchloride (PVDC) produced by polymerization of vinylidendichloride CH₂=CCl₂ (1,1-Dichlorethen) as monomer; polymonochlorotrifluoroethylene (PCTFE) produced by polymerization of 1-Chlor-1,2,2-trifluorethen CClF=CF₂ as monomer; polyvinylidene fluoride (PVDF) produced by polymerization of vinylidene fluoride CH₂=CF₂ (1,1-difluoro ethylene) as monomer, as well as copolymers produced by polymerization of said monomers and optionally additional copolymerizable monomers.

In particular, suitable halogenated polyolefins, such as PVC, are phthalate free, e.g. phthalate free PVC (non-DEHP-PVC-tubing).

Preferably, the at least one halogenated polyolefin is selected from polyvinylidene fluorides (PVDF) and polymonochlorotrifluoroethylenes (PCTFE). For example, PCTFE is commercially available as NEOFLON^{™} PCTFE from Daikin Industries, Aclar^{®} from Honeywell, or VOLTALEF^{®} from Arkema Group.

Polyvinylidene fluorides (PVDF) are commonly known, often semi-crystalline, thermoplastic fluoroplastics, which are obtained via polymerization of vinylidene fluoride CH₂=CF₂ (1,1-difluoro ethylene), optionally together with suitable comonomers. Typically, PVDF may be transparent in thin layers and appears milky white at higher thickness. Generally, PVDF is synthesized by the free radical polymerization in suspension or emulsion under controlled conditions of pressure and of temperature, e.g. at a temperature from 10-150°C and pressure of 10-300 atm. For example, PVDF is often used for the production of films or sheets.

Preferably, the PVDF, used as thermoplastic polymer A, may be selected from vinylidene fluoride homopolymers, as well as copolymers or terpolymers of vinylidene fluoride, wherein typically the amount of vinylidene fluoride units is at least 50 wt.-%, preferably at least 60 wt.-%, more preferably at least 70 wt.-%, based on the total weight of all monomer units in the PVDF. For example co- and/or terpolymers of vinylidene fluoride may be obtained by polymerization of vinylidene fluoride together with one or more comonomers, selected from partly or fully fluorinated olefins, such as vinyl fluoride, trifluoro ethylene, tetrafluoro ethylene, 3,3,3-trifluoro-1-propylene, 1,2,3,3,3-pentafluoropropylene, 3,3,3,4,4-pentafluoro-1-butylene, hexafluoro propylene, and hexafluoro isobutylene; partly or fully chlorinated fluoro-olefins, such as chlorotrifluoroethylene; perfluorinated vinyl ethers, such as perfluoro methyl vinyl ether, perfluoro ethyl vinyl ether, perfluoro-n-propyl vinyl ether, and perfluoro-2-propoxypropyl vinyl ether; other fluorine-containing monomers, such as fluorovinyl sulfonic acid; olefins, such as ethylene or propylene.

According to a preferred embodiment the thermoplastic polymer A comprises or consists of PVDF, wherein the PVDF is selected from homopolymers of vinylidene fluoride, and copolymers of vinylidene fluoride with one or more comonomer selected from vinyl fluoride, trifluoro ethylene, tetrafluoro ethylene, hexafluoro propylene, hexafluoro isobutylene, chlorotrifluoro ethylene, perfluoro methyl vinyl ether, and fluorovinyl sulfonic acid; more preferably selected from tetrafluoro ethylene, hexafluoro propylene, and chlorotrifluoro ethylene.

The mass average molecular weight of the PVDF used as thermoplastic polymer A is preferably 50,000 to 450,000; more preferably 100,000 to 400,000; and even more preferably 110,000 to 300,000. Typically, the mass average molecular weight of the PVDF can be measured via gel permeation chromatography (GPC), using dimethyl formamide as a solvent and calibration using polystyrene standard.

For example, PVDF as described in EP 2046888, US 2016/0200884 A1and WO 2009/000566 can be used as thermoplastic polymer A in the present invention.

Typically, for the purposes of the invention commercially available grades of PVDF may be utilized, such as Kureha KF polymers from Kureha Corporation, Japan (e.g. KF TH850, KF TH1000, and KF TH1100), Kynar^{®} grades from Arkema (e.g. Kynar^{®} 760, Kynar^{®} 740, Kynar^{®} 720, and Kynar^{®} 710), 3M^{®} Dyneon^{®} grades from Dyneon, and Solef^{®} grades from Solvay (e.g. Solef^{®}1006, 1008, 1015, 5140, 6008, 6010, 6012, 60512, 11008, 21508,11010, 21510).

### Thermoplastic polymer material

The thermoplastic polymer material comprises the thermoplastic polymer(s) A as described above and optionally additional components, such as one or more polymeric components B different from thermoplastic polymer A, one or more plasticizers C, and/or one or more further components D, preferably selected from additives, auxiliaries and/or fillers.

In a preferred embodiment the thermoplastic polymer material comprises (preferably consists of), based on the total weight of the thermoplastic polymer material:
A. 50.0 to 100.0 wt.-%, preferably 54.0 to 93.0 wt.-%, of at least one thermoplastic polymer A, selected from cyclic polyolefins, such as cyclic olefin polymer COP, cyclic olefin copolymer COC and cyclic block copolymer CBC; aliphatic polyamides, aliphatic polyurethanes and halogenated polyolefins, more preferably selected from cyclic olefin polymer COP, cyclic olefin copolymer COC and cyclic block copolymer CBC;
B. 0.0 to 50.0 wt.-%, preferably 1.0 to 40.0 wt.-%, of at least one polymeric component B, different from A,
C. 0.0 to 20.0 wt.-%, preferably 1.0 to 10.0 wt.-%, of at least one plasticizer C, e.g. selected from polyethylene glycol having a molecular weight from 500 to <10 000 g/mol, fatty acids, fatty acid esters, tributyl citrate, butyl lactate, and 1,2-cyclohexane dicarboxylic acid diisononyl ester; and
D. 0.0 to 10.0 wt.-%, preferably 0.0 to 5.0 wt.-%, of at least one further component D, preferably selected from additives, auxiliaries and/or fillers.

According to the present invention the thermoplastic polymer material has a transmittance of at least 10 %, preferably at least 15 %, more preferably at least 20 %, also preferably at least 25 %, most preferably at least 30 %, averaged over the wavelength range from 260 nm to 300 nm, preferably from 260 to 280 nm, and measured in accordance with ISO 13468-2 at a thickness of 3 mm.

Further preferably, the thermoplastic polymer material has a transmittance of at least 8 %, preferably at least 10 %, also preferably at least 15 %, more preferably at least 25 %, at a wavelength of 265 nm, measured in accordance with ISO 13468-2 at a thickness of 3 mm.

Further preferably, the thermoplastic polymeric material has a total transmittance of at least 10 %, preferably at least 20 %, more preferably at least 30 %, integrated over the wavelength range from 260 to 300 nm, and measured in accordance with ISO 13468-2 at a thickness of 3 mm.

Generally, the transmittance of the thermoplastic polymer material at the wavelength of 265 nm or in the range of 260 to 300 nm can be measured using an instrument as defined in the standard ISO 13468-2 which is suitable for measurements in said wavelength range (e.g. a Varian Cary 5000). Generally, the transmittance of a material is defined as the ratio of transmitted optical power to the incident optical power for a given thickness, wherein the light resulting from directed transmission as well as from diffuse transmission is included.

Preferably, the thermoplastic polymer material and/or the formed article made thereof are transparent in the visible range of electromagnetic spectrum. In terms of the present invention, "*a transparent polymer material*" or *"transparent formed article"* means that the polymer material or the formed article has a haze according to standard ASTM D1003 of less than 50 %, preferably less than 40 %, more preferably less than 30 %, most preferably less than 20 %, measured at 23 °C on an injection molded specimen having a thickness of 3 mm.

The thermoplastic polymer material can be prepared by dry blending the components, which may be present as powder, particles or preferably pellets. The thermoplastic polymer material can also be prepared by mixing the components either at the same time or successively into the melt of the thermoplastic polymer A, optional in the melt of thermoplastic polymer A and optional polymeric component B. The thermoplastic polymer material can also be prepared by melting and mixing the individual components in the molten state or by melting dry premixes of the individual components to give a ready-to-use molding material. This can be effected, for example, in single-screw or twin-screw extruders. The extrudate obtained can then be granulated. Customary additives, auxiliaries and/or fillers can be directly admixed or added later by end users as required.

In particular, the thermoplastic polymer material can be processed via commonly known thermal molding processes, e.g. via injection molding, to produce formed articles having various arbitrary shape.

In particular, the present invention is directed to a method for UV sterilization of a formed article, wherein the formed article is selected from medical devices, containers and/or packages used for cosmetic or pharmaceutical products or used in food industry, for example in production and packaging of beverages, meat products, or dairy products.

### Polymeric component B

In a preferred embodiment the thermoplastic polymer material comprises 0.0 to 50.0 wt.-%, preferably 1.0 to 40.0 wt.-%, more preferably 5.0 to 35.0 wt.-%, also preferably 10.0 to 30.0 wt.-%, also preferably from 10.0 to 50.0 wt.-%, based on the total polymer material, of one or more polymeric components B, which are different from A and optionally C and D. In particular the optional polymeric component B is compatible, i.e. homogenous miscible, with the thermoplastic polymer A and may form a homogenous polymer matrix together with the thermoplastic polymer A, and optional the components C and/or D. More preferably, said polymer blend (respectively polymer matrix) is transparent in the visible range of electromagnetic spectrum.

In particular, the optional polymeric component C is selected, so that it increases or does not significantly lower the UV transmittance of the thermoplastic polymer material in the desired range, e.g. from 260 to 300 nm, preferably from 260 to 280 nm, more particularly at 265 nm.

Preferably, the optional polymeric component C is selected from polyolefins, such as polyethylene and polypropylene; alkyl(meth)acrylate polymers, polycarbonates, silicones and/or higher polyethylene glycols (PEG), e.g. polyethylene glycols having a molecular weight of at least 10,000 g/mol, preferably in the range of 10,000 to 200,000 g/mol.

Typically, a suitable alkyl(meth)acrylate polymer may comprise, based on the total weight of the alkyl(meth)acrylate polymer:
70.0 to 100.0 wt.-%, preferably from 80.0 to 100.0 wt.-%, more preferably from 90.0 to 99.9 wt.-%, of at least one alkyl methacrylate monomer having from 1 to 20, preferably from 1 to 12, more preferably from 1 to 8, most preferably from 1 to 4, carbon atoms in the alkyl radical, and
0.0 to 30.0 wt.-%, preferably from 0.0 to 20.0 wt.-%, more preferably from 0.1 to 10.0 wt.-%, of at least one alkyl acrylate monomer, having from 1 to 20, preferably from 1 to 12, more preferably from 1 to 8, in particular from 1 to 4, carbon atoms in the alkyl radical.

According to one embodiment, cyclic polyolefins, such as cyclic olefin polymer COP, cyclic olefin copolymer COC and cyclic block copolymer CBC are used as thermoplastic polymer A in combination with at least one linear polyolefin, such as polyethylene, polypropylene and copolymers of ethylene and propylene as optional polymeric component C, wherein both polymeric components are melt blended to form a homogeneous blend, using appropriate mixing ratios.

According to another preferred embodiment the polymeric component C comprises (preferably consists of) higher polyethylene glycols (PEG). Polyethylene glycols (PEG) are commonly known hydrophilic, biocompatible polymers, which are often used in medical application. Generally, PEG is prepared by a ring-opening polymerization of ethylene oxide. Typically, PEGs are available in a broad range of molecular weights and molecular weight distribution (from polydisperse to discrete PEGs). As a rule, low molecular weight PEG, e.g. PEG having a molecular weight less than 10,000 g/mol, are commonly known plasticizers, e.g. utilized as component D. Preferably, polyethylene glycol (also referred to as polyethylene oxide) used as polymeric component C has a molecular weight of at least 10,000 g/mol, preferably from 10,000 to 200,000 g/mol, more preferably from 10,000 to 100,000 g/mol. Typically, the molecular weight of PEGs refers to the weight averaged molecular weight.

Typically, for the purposes of the invention commonly known, commercially available grades of PEG may be utilized, for example PEG 10,000 or PEG 20,000, wherein the number indicates the molecular weight, typically given as weight average molecular weight.

Typically, the thermoplastic polymer A and PEG, as well as thermoplastic polymer A, PEG, and optional polymeric component C, can be melt blended to form a homogeneous blend, using appropriate mixing ratios.

### Plasticizer C

In a preferred embodiment the thermoplastic polymer material comprises 0.0 to 20.0 wt.-%, preferably 0.0 to 10.0 wt.-%, more preferably 0.0 to 5.0 wt.-%, also preferably 0.1 to 20.0 wt.-%, also preferably from 1.0 to 10.0 wt.-%, based on the total polymer material, of one or more plasticizers C.

Typically, ductility, craze resistance and/or chemical resistance of the thermoplastic polymer material and of the formed article made thereof can be improved by the addition of one or more appropriate plasticizers as component C. Plasticizers per se are familiar to the skilled person and described for example in Ullmann's Encyclopaedia of Industrial Chemistry, 2012, Plasticisers, D. F. Cadogan etc. For the purpose of the present invention, the plasticizers C usually have a molecular weight of less than 10,000 g/mol, and a melting temperature of not more than 40 °C.

If a polymeric compound is used as a plasticizer in the thermoplastic polymer material, such polymeric compound should ideally have a glass transition temperature T_{g} of not more than 40 °C, as measured according to standard ISO 11357-2:2013 and/or an weight averaged molecular weight of not more than 200,000 g/mol, preferably less than 10,000 g/mol.

Furthermore, to ensure that presence of the plasticizers does not adversely affect optical properties of the thermoplastic polymer material, the plasticizer should be miscible with the thermoplastic polymer material.

Examples of particularly suitable plasticizers include in particular polyethylene glycol having a molecular weight from 500 to <10,000 g/mol, fatty acids, fatty acid esters, tributyl citrate, butyl lactate, and 1,2-cyclohexane dicarboxylic acid diisononyl ester (commercially available as a mixture of isomers under the trademark name Hexamoll^{®} DINCH from BASF SE, Ludwigshafen, Germany). Typically, the fatty acids or fatty acid esters encompass fatty acid alkyl chains having C1-C30, preferably C4-C26, carbon atoms, wherein the alkyl chain can be linear or branches, saturated or unsaturated. Typically, the fatty acid esters are C1-C30 alkyl triglycerides. 1,2-cyclohexane dicarboxylic acid diisononyl ester is typically a mixture of isomers and usually comprises 10 wt.-% *n-*nonylalcohol, 35 to 40 wt.-% methyloctylalcohol, 40 to 45 wt.-% dimethylheptylalcohol und 5 to 10 wt.-% methylethylhexylalkohol, based on the total weight of isononyl alcohol residues.

In a preferred embodiment, the thermoplastic polymer material comprises 0.01 to 20.0 wt.-%, preferably from 0.1 to 15.0 wt.-%, more preferably from 1.0 to 10.0 wt.-%, based on the total polymer material, of at least one polyethylene glycol having a molecular weight from 500 to <10,000 g/mol, as plasticizer C.

### Further component D

The thermoplastic polymer material utilized in the inventive method for UV sterilization may comprise an optional further component D, which is different from components A, B, and C. For example, the further component D may be selected from non-polymeric components.

Typically, the further component D is selected from common additives, such as impact-modifiers, flow improvers, stabilizers, mold release agents, processing aids, lubricants, anti-static agents, antimicrobial agents, colorants, coloring agents, and dyes, in usual amounts, in order to adjust mechanical and/or optical properties, as long as the transmittance of the polymeric materials in the relevant range of wavelength, i.e. 260 nm to 300 nm, preferably at 265 nm, is not less than 10%.

Furthermore, the optional further component D may include an impact modifier, e.g. a particulate impact modifier which is dispersed the polymer matrix comprising the thermoplastic polymer A. Preferably, the thermoplastic polymer material may comprises 0.0 to 40.0 wt.-%, preferably 0.0 to 35.0 wt.-%, also preferably 5.0 to 30.0 wt.-%, based on the total polymer material, of at least one impact modifier as component D. In case that an impact modifier is present in the thermoplastic polymer material the amount of the least one thermoplastic polymer A is adapted accordingly, in particular the of the least one thermoplastic polymer A is present in an amount of at least 50.0 wt. Typically, impact strength, craze resistance and/or chemical resistance can be improved by the addition of one or more impact modifiers. Preferably, the optional impact modifier as component D is selected, so that it does not significantly lower the UV transmittance of the thermoplastic polymer material in the desired range, e.g. from 260 to 300 nm, preferably from 260 to 280 nm, more particularly at 265 nm.

For example, the optional impact-modifier used as component D is selected from particulate multiphase graft copolymers (also referred to as graft copolymer in the following) having a core-shell structure including at least one core and at least one shell, e.g. core-shell structure or core-shell-shell structure.

Preferably, particulate multiphase graft copolymers may comprise a core and at least one shell, wherein typically the outer shell represents a hard phase comprising at least thermoplastic polymer A. Typically, the particulate multiphase graft copolymer comprises an elastomeric core or at least one elastomeric intermediate layer, wherein the elastomeric phase (also referred to as soft core or soft layer) typically is cross-linked and has a glass transition temperature T_{g} < 20 °C, preferably T_{g} < 0°C, for example determined in a known manner, such as differential scanning calorimetry (DSC). The glass transition temperature T_{g} may also be calculated as an approximation by means of the Fox equation.

Typically, the further component D, in particular selected from common non-polymeric additives, may be present in an amount of 0.0 to 15.0 wt.-%, preferably 0.0 to 10.0 wt.-%, more preferably 0 to 5 wt.-%. Preferably, the further component E may be present in an amount of 0.0001 to 5.0 wt.-%, also preferably 0.001 to 2.0 wt.-%, based on the total polymer material.

Preferably, the thermoplastic polymer material may comprise as optional further component D, one or more additive selected from external lubricants, antioxidants, flame retardants, further hindered amine light stabilizers (HALS), flow improvers such as stearyl alcohol or palmitic acid, metal additives for screening against electromagnetic radiation, antistatic agents, mould release agents, dyes, pigments, adhesion promoters, anti-weathering agents, heat stabilizers, UV stabilizers, UV absorbers, gamma ray stabilizers, antioxidants, and fillers, provided that the UV transmittance of the thermoplastic polymer material in the relevant range is not adversely affected by these additives.

Lubricants and mold release agents which can reduce or completely prevent possible adhesion of the molding material to the injection mold are important for the injection molding process and may preferably be employed.

For example, lubricants, selected from the group consisting of saturated fatty acids having less than 20, preferably 16 to 18, carbon atoms or of the saturated fatty alcohols having less than 20, preferably 16 to 18, carbon atoms, may be present as auxiliaries. For example, stearic acid, stearyl alcohol, palmitic acid, palmitic alcohol, lauric acid, lactic acid, glycerol monostearate, pentaerythrol, and industrial mixtures of stearic and palmitic acid. Also suitable are n-hexadecanol, n-octadecanol and industrial mixtures of *n-*hexadecanol and n-octadecanol. A particularly preferred lubricant or mold release agent is stearyl alcohol.

The lubricants are typically used in amounts of not more than 0.35 wt.-%, for example 0.05 wt.-% to 0.25 wt.-% based on the weight of the thermoplastic polymer material. In a preferred embodiment, at least one sterically hindered amine may be used as component D, giving an improvement in resistance to weathering, yellowing or degradation of the polymer material. Especially preferred sterically hindered amines include dimethylsuccinate-1-(2-hydroxyethyl)-4-hydroxy-2,2,6,6-tetramethylpiperazine polycondensate, poly[{6-(1,1,3,3-tetramethylbutyl)amino-1,3,5-triazine-2,4-diyl}{(2,2,6,6-tetramethyl-4-piperidyl)imino}-hexamethylene{(2,2,6,6-tetramethyl-4-piperidyl)imino}], *N*,*N*'-bis(3-aminopropyl)ethylene-diamine-2,4-bis[*N*-butyl-*N*-(1,2,2,6,6-pentamethyl-4-piperidyl)amino]-6-chloro-1,3,5-triazine condensate, bis(2,2,6,6-tetramethyl-4-piperidyl)sebacate and 2-(3,5-di-t-4-hydroxybenzyl)-2-n-butylmalonate bis(1,2,2,6,6-pentamethyl-4-piperidyl).

In a preferred embodiment, the thermoplastic polymer material is free of any UV absorber and/or UV stabilizer. Further, it is possible, that the amount of UV absorber and/or UV stabilizer is less than 100 ppm, preferably less than 10 ppm, based on the thermoplastic polymer material.

However, it is possible that the thermoplastic polymer material comprises a small amount of specific UV absorbers, that do not significantly reduce the transmittance at the desired UV wavelength range, e.g. from 260 to 300 nm, preferably from 260 to 280 nm, more particularly at 265 nm. Typically, the thermoplastic polymer material may comprise up to 0.3 wt.-%, preferably up to 0.1 wt.-%, for example 0.0001 to 0.1 wt.-%, preferably from 0.005 to 0.05 wt.-%, based on the weight of the thermoplastic polymer material of specific UV absorbers as component D.

Suitable optional UV absorbers may be, for example, derivatives of benzophenone (e.g. 2-hydroxy-4-n-octyloxybenzophenone, 2,4-dihydroxybenzophenone, 2,2'-dihydroxy-4-methoxybenzophenone, 2,2',4,4'-tetrahydroxybenzophenone, 2,2'-dihydroxy-4,4'-dimethoxybenzophenone, 2-hydroxy-4-methoxybenzophenone), benzotriazoles (e.g. 2-(2'-hydroxy-5'-methyl-phenyl)benzotriazole, commercially available as Tinuvin^{®} P, from BASF SE; Ludwigshafen, Germany, or 2-(2'-hydroxy-3'-dodecyl-5'-methyldecyl)benzotriazole, substituted benzotriazoles (z.B. 2-(2-hydroxy-5-methylphenyl)benzotriazole, 2-[2-hydroxy-3,5-di(alpha,alphadimethylbenzyl)phenyl]benzotriazole, 2-(2-hydroxy-3,5-di-*tert*.-butylphenyl)benzotriazole, 2-(2-hydroxy-3,5-di-*tert*.-amylphenyl)benzotriazole, 2-(2-hydroxy-5-*tert*.-butylphenyl)benzotriazole, 2-(2-hydroxy-3-sec-butyl-5-*tert*.-butylphenyl)benzotriazole and 2-(2-hydroxy-5-*tert*.-octylphenyl)benzotriazole). 2-(2'-hydroxy-5'-methylphenyl)benzotriazole (commercially available as Tinuvin^{®} P) , is particularly preferred due its low absorbance in the UV-C range. In a preferred embodiment, the thermoplastic polymer material comprises 0 to 0.3 wt.-%, preferably 0 to 0.1 wt.-%, also preferably from 0.0001 to 0.1 wt.-%, more preferably from 0.005 to 0.05 wt.-%, based on the weight of the thermoplastic polymer material, of at least one benzotriazole type UV absorber, more preferably 2-(2'-hydroxy-5'-methyl-phenyl)benzotriazole (Tinuvin^{®} P) as component D.

Other suitable UV absorbers may be, for example, oxanilide and commonly known derivatives thereof, for example N-(2-ethoxyphenyl)-N'-(2-ethylphenyl) ethane diamide, which is commercially available from BASF SE as Tinuvin^{®} 312.

Another suitable UV absorber is selected from known sterically hindered phenolic compounds, for example octadecyl-3-(3,5-di-tert-butyl-4-hydroxyphenyl)propionate, which is commercially available from BASF SE under the product name IRGANOX^{®} 1076.

As will be readily appreciated by a skilled person, mixtures of chemically different UV absorbers may also be employed.

Examples of suitable free radical scavengers/UV stabilizers include inter alia sterically hindered amines, which are known by the name HALS ((Hindered Amine Light Stabiliser) (for example described in Kunststoffe [Plastics], 74 (1984) 10, pages 620 to 623; Farbe + Lack [Paints + Finishes], 96th year, 9/1990, pages 689 to 693). Often, such sterically hindered amines do not absorb in the UV range. They trap free radicals formed, which once again the UV absorbers are incapable of doing. Examples of suitable HALS stabilizers are bis(2,2,6,6-tetramethyl-4-piperidyl) sebacate, 8-acetyl-3-dodecyl-7,7,9,9-tetramethyl-1,3-8-triazaspiro(4,5)decane-2,5-dione, bis(2,2,6,6-tetramethyl-4-piperidyl)succinate, poly(N-ß-hydroxyethyl-2,2,6,6-tetramethyl-4-hydroxypiperidine succinic acid ester) and bis(N-methyl-2,2,6,6-tetramethyl-4-piperidyl) sebacate, and mixtures thereof.

The free radical scavengers / UV stabilizers can be used in the thermoplastic polymer material in amounts of 0.01 to 1.5 wt.-%, especially in amounts of 0.02 to 1.0 wt.-%, in particular in amounts of 0.02 to 0.5 wt.-%, based on the total polymer material.

### Properties of the thermoplastic polymer material

As already mentioned, the thermoplastic polymer material or the formed article made thereof can advantageously be sterilized, including sterilization of the inner surface, using UV radiation having a wavelength in the range of 260 nm to 300 nm, more preferably using UV radiation having a wavelength of 265 nm.

The transmittance of the thermoplastic polymer material at the wavelength of 265 nm or in the range of 260 to 300 nm is measured using an instrument as defined in the standard ISO 13468-2 which is suitable for measurements at this wavelength (e.g. a Varian Cary 5000 spectrophotometer). For the measurement a test plaque of the following dimensions can be used: 2 × 3 inches (5.08 × 7.62 cm), thickness of 0.125 inch (3.175 mm).

Additionally, the thermoplastic polymer material or the formed article made thereof has an excellent transparency and a substantially non-cloudy appealing appearance. In particular, haze of the thermoplastic polymer material measured at 23 °C on an injection molded specimen having a thickness of 3 mm according to standard ASTM D1003 (2013) is lower than 50%, preferably lower than 40%, more preferably lower than 30% even more preferably lower than 20 %.

Furthermore, the thermoplastic polymer material preferably shows a light transmittance, TD65 according to DIN 5033 - 7 (2014) of at least 80 %, preferably at least 90 %, measured at 23 °C on an injection molded specimen having a thickness of 3 mm.

The Vicat softening temperature of the thermoplastic polymer material according to ASTM D-1525, (Method B, 5.0 kg, 60 °C/hr or FT/DS Correlation) is advantageously at least 80 °C, preferably at least 90°C, more preferably at least 100 °C.

The nominal elongation at break of the thermoplastic polymer material according to ASTM D-638 should preferably be at least 3.0 %, particularly preferably at least 4.0 %.

Due to its advantageous rheological properties, the thermoplastic polymer material as described is highly suitable for manufacturing of medical grade articles by means of injection molding or other suitable thermoforming process. The thermoplastic polymer material typically has a melt volume flow rate MVR measured according to ASTM D-1238 at 260 °C and 2.16 kg, of greater than 0.5 cm³/10 min, preferably greater than 1.0 cm³/10 min, more preferably greater than 3 cm³/10 min.

### UV exposure (step b)

The inventive method for UV sterilization encompasses the exposure of the outer surface of the formed article with UV radiation at a wavelength in the range of 260 to 300 nm, preferably with UV radiation at a wavelength of 265 nm, wherein at least one part of said formed article is made from (meaning consists of) an thermoplastic polymer material as described, and wherein the UV radiation is typically provided by one or more appropriate UV radiation source, preferably selected from UV LEDs.

Typically, the exposure with UV radiation is carried out in any suitable equipment that allows an appropriate arrangement of the UV radiation source and the formed article that is sterilized. If necessary, such equipment may include a suitable housing made from a non-UV transmissive material in order to avoid undesirable UV exposure to the environment.

Particularly, exposure of the formed article with UV radiation in the range of 260 to 300 nm means that the at least one part of the formed article, which is made of the thermoplastic polymer material, is exposed to said UV radiation.

In a preferred embodiment the whole formed article is made from the thermoplastic polymer material as described, and the UV sterilization step b) encompasses the exposure of the whole formed article or of at least a part of the formed article with UV radiation in the range of 260 nm to 300 nm.

Typically, the inventive method may also encompass the sterilization of the outer surface of the formed article and/or of parts that are not made of the thermoplastic polymer material caused by the UV radiation. However, the main advantage of the inventive method is that effective sterilization of the inner surface of the formed article, which is not directly exposed to the UV radiation, is achieved.

Preferably, the desired reduction or inhibition of growth of microorganisms, for example as described below, is achieved by the inventive method for UV sterilization. Further, it is possible to combine the inventive method for UV sterilization with one or more commonly known, physical or chemical processes of sterilization, disinfection and/or decontamination, such as chemical sterilization and/or disinfection using commonly known disinfectants, e.g. ethylene oxide, vaporized hydrogen peroxide, ozone, alcohol/water mixtures; irradiation with gamma radiation, electron beam or X-ray; heat and/or steam treatment.

Furthermore, according to another preferred embodiment of the invention it is possible that the formed article, preferably a medical device, more preferably a disposable medical device, is surrounded by an UV-transmissive material, e.g. a polymeric protective packaging, during the UV exposure step b. In particular *"UV transmissive"* means that the material has a transmittance of at least 10 %, averaged over the wavelength range from 260 nm to 300 nm, and measured in accordance with ISO 13468-2 at a thickness of 3 mm. In a preferred embodiment, the formed article, preferably a medical device, more preferably a disposable medical device, is surrounded by a protective packaging during the UV exposure step b, wherein the protective packaging is made of an UV-transmissive polymer material, preferably selected from alkyl(meth)acrylate polymers, polyethylene PE, polypropylene PP, polyvinylchloride PVC (in particular PVC without phthalate plasticizer, such as di(2-ethylhexly)phthalate DEHP, referred to as non-DEHP-PVC), polysiloxane (silicone), and thermoplastic polymer material as described in the present invention, such as cyclic polyolefins; aliphatic polyamides, aliphatic polyurethanes and halogenated polyolefins.

Preferably, such protective packaging is impervious to microorganisms.

It is also possible that the formed article is placed in a container made of UV transmissive quartz glass during UV exposure. Further, the formed article, preferably a medical device, more preferably a medical device surrounded by a protective packaging, is placed in a second outer packaging/container after UV exposure.

### UV radiation source

The choice of the ultraviolet (UV) radiation source for use in the inventive method is not particularly limited, as long as the UV radiation source emits UV radiation at a wavelength in the range of 260 nm to 300 nm, preferably 260 to 280 nm. It is preferred but not necessary that at least a significant amount of emission spectrum of the UV source, preferably selected from UV light emitting diodes (UV-LEDs), is in the range of 260 nm to 300 nm, preferably 260 to 280 nm. More preferably, the UV source has at least one emission peak falling within said range.

The emission peak half width of the UV radiation source is typically not higher than 30 nm, preferably not higher than 20 nm, more preferably not higher than 15 nm. Preferably, the operating temperature of the UV radiation source is compatible with the employed polymeric material. Ideally, the UV radiation source is substantially monochromatic i.e. has a single emission peak. The examples of suitable UV radiation source include a UV light emitting diode (UV-LED), an excimer laser, a plasma or synchrotron source or a gas discharge tube.

Typically, UV mercury lamps only emit light at 254 nm, so it is preferred to use UV light-emitting diodes (UV-LEDs) which can be configured to emit certain target wavelengths, i.e. desired UV wavelength from 260 to 300 nm, preferably from 260 to 280 nm, more particularly at 265 nm, in the inventive method. Further, UV mercury lamps have additional disadvantages, such as a long warm-up time, and risk of mercury exposure. Thus, the use of UV-LEDs is particularly preferred. In a particularly preferred embodiment, one or more UV-C LEDs are used as UV radiation source in the inventive method for UV sterilization. It is advantageous to use UV-C LEDs as it allows adjusting and combination of the most effective wavelengths in view of the thermoplastic polymer material as well as target microorganisms. Generally, each microorganism requires a specific UV dosage depending on the UV wavelength and based on the desired log reduction.

In some embodiments of the present invention, the UV radiation source is a pulsed UV radiation source, e.g. a pulsed UV-LED.

Particularly, the UV radiation source is selected from commonly known and commercially available germicidal UV light sources, e.g. germicidal UV-LEDs, showing emission, particularly an emission peak, in the desired wavelength range, e.g. from 260 to 300 nm, preferably from 260 to 280 nm, more particularly at 265 nm. Ideally, the emission spectrum of the UV radiation source comprises an emission peak or a single emission peak in the range of 260 nm to 300 nm, preferably at 265 nm. For example, these LEDs are commercially available as Klaran^{®} UV-C LEDs from Crystal IS Inc. (USA); as UV-C-LED having a wavelength of 265 nm from Stanley Electric Co.; or as Oslon UV 3636 from Osram Opto Semiconductors GmbH.

Preferably, one or more UV LEDs, selected from UV LEDs emitting at 266 nm, 270 nm, 275 nm, 279 nm, 280 nm, or 285 nm, were utilized in the inventive method. More preferably at least one UV LED emitting at 265 nm were utilized in the inventive method. Generally, the UV irradiance or also referred to as UV intensity (given in W/m² or mW/cm²) refers to the irradiance field of an UV irradiation system including the UV radiation source and the surface exposed to said UV radiation, i.e. means the total radiant flux (energy) incident on a surface from all directions. The UV irradiance greatly depends on the distance from the UV source and the transmittance of the medium. Preferably, the UV irradiance in the inventive method for sterilization provided by the UV radiation source, preferably by one or more UV LEDs, is in the range of 0.1 to 1.0 mW/cm², preferably 0.2 to 0.5 mW/cm² (relating to the outer surface of the formed article).

Generally, the UV dose (given in mJ/cm² or J/m²) refers to the amount of UV irradiation incident on a surface or in particular absorbed by an exposed population of microorganisms. The UV dose results from the UV irradiance of the UV radiation source and the exposure time (in seconds). Generally, the required UV dose depends on the UV wavelength range used, the target microorganism(s) and the required reduction of growth or number of microorganisms, which is typically given in CFU log reductions.

Preferably, the exposure of the outer surface of the formed article with UV radiation is carried out in such way that a UV dose in the range of 1 to 10,000 mJ/cm², preferably 2 to 2,000 mJ/cm², also preferably 10 to 1,000 mJ/cm² is effected (relating to the outer surface of the formed article). It was found that such UV dose is often suitable to effect the desired reduction of microorganism at the inner surface of a formed article, wherein the formed article has a thickness of less than 3.0 mm, preferably less than 1.0 mm, more preferably less than 0.5 mm, for example in the range from 0.01 to 4 mm, preferably in the range from 0.1 to 1 mm.

Typically, in the inventive method for UV sterilization the distance between the UV radiation source and the outer surface of the formed article is adjusted so that the desired sterilization effect, i.e. the desired CFU log reduction, is obtained.

Typically, the UV radiation exposure is carried out one or several times, each cycle is about 10 to 600 seconds, preferably 60 to 600 seconds. Typically, the exposure time is selected depending on the desired sterilization effect, i.e. the desired CFU log reduction, and/or the UV radiation source.

### Reduction of microorganisms

Generally, sterilization results in colony forming units (CFU) log reduction ≥ 6 (i.e. reduction of ≥10⁶ CFU) of present microorganisms, including the most resistant spores. Generally, disinfection results in CFU log reduction ≥ 3 of present microorganisms, not including spores. Generally, decontamination achieves minimum log reduction of ≥1 CFU of microorganisms. In terms of the present invention the term *"sterilization"* or *"method for sterilization"* includes sterilization, disinfection and/or decontamination.

According to a preferred embodiment, the number of colony forming units (CFU) of one or more relevant microorganisms, for example mentioned below, is reduced/inhibited by at least 1 log, preferably at least 2 log, preferably at least 3 log, preferably at least 4 log, also preferably at least 5 log, even more preferably of at least 6 log, using the inventive method for UV sterilization. As a skilled person known, the desired log reduction can for example be obtained by adjusting exposure time and/or UV dose.

Generally, the term microorganisms include bacteria, fungi, algae, viruses, archaea, protozoa. Preferably, the inventive method for UV sterilization is directed to reduction of microorganisms selected from bacteria, fungi (in particular yeast), and viruses, more preferably selected from bacteria and/or yeast.

Typically, the inventive method for UV sterilization can inhibit the growth of one or more microorganisms, which is relevant for the specific intended use of the formed article, e.g. use as medical device or as food container. For example, the inventive method for UV sterilization may inhibit the growth of one or more microorganisms, for example of genus selected from *Escherichia, Salmonella, Listeria, Aspergillus, Bacillus, Cryptosporidium, Clostridium, Streptomyces, Aeromonas, Candida, Helicobacter, Klebsiella, Legionella, Listeria, Pseudomonas, Staphylococcus, Streptococcus, Lactobacillus, Bifidobacterium, Oenococcus, and Saccharomycodes.*

For example, the inventive method for UV sterilization may inhibit the growth of one or more species of microorganisms, selected from *Escherichia coli, Salmonella Typhimurium, Listeria monocytogenes, Cryptosporidium, Aspergillus niger, Bacillus anthracis, Bacillus cereus, Bacillus megaterium, Bacillus subtilis, Clostridium pasteurianum Streptomyces griseus, Aeromonas hydrophila, Candida auris, candida , Helicobacter pylori, Klebsiella pneumoniae, Legionella pneumophila, Listeria monocytogenes, Pseudomonas aeruginosa, Staphylococcus aureus, Streptococcus hemolyticus, Oenococcus oeni, Lactobacillus acidophilus, Lactobacillus plantarum, Bifidobacterium lactis, Bifidobac bifidum, Lactobacillus rhamnosus, and Bifidobacterium breve.*

Typically, in the inventive method for UV sterilization a CFU log reduction of up to 4 can be obtained for microorganism *Escherischia coli* using an UV LED having emission peak at 265 nm utilizing a UV dose in the range of 3 to 20 mJ/cm², wherein the CFU log reduction is effected at the inner surface of the formed article.

### Formed article

In another aspect, the present invention is directed to a formed article which is sterilized in the inventive method for UV sterilization, preferably selected from medical devices; containers and/or packages used for cosmetic or pharmaceutical products or used in food industry, wherein at least one part of said formed article is made from an thermoplastic polymer, which comprises at least 50 wt.-%, preferably at least 60 wt.-%, more preferably at least 70 wt.-%, based on the thermoplastic polymer material, of at least one thermoplastic polymer A, selected from cyclic polyolefins, such as cyclic olefin polymer (COP), cyclic olefin copolymer COC and cyclic block copolymer CBC; aliphatic polyamides, aliphatic polyurethanes and halogenated polyolefins; and wherein the thermoplastic polymer material has a transmittance of at least 10 %, preferably at least 15 %, more preferably at least 20 %, also preferably at least 25 %, averaged over the wavelength range from 260 nm to 300 nm, and measured in accordance with ISO 13468-2 at a thickness of 3 mm. Preferably, the inventive formed article comprises at least one inner surface, that includes openings, cavities and/or pores, which cannot or only with high difficulties be exposed to UV radiation directly.

Said formed articles can advantageously be sterilized by the inventive method for UV sterilization as described above. The description and the preferred embodiments of the inventive method for UV sterilization apply to the inventive formed article accordingly.

Preferably, the formed article or the at least one part of the formed article made of the thermoplastic polymer material has a wall thickness of less than 3.0 mm, preferably less than 1.0 mm, more preferably less than 0.5 mm, for example in the range from 0.01 to 4 mm, preferably in the range from 0.1 to 1 mm. It was found that UV transmittance through the thermoplastic polymer material as described is high enough in order to effectively reduce the growth of microorganisms at the inner surface of the formed article.

In a preferred embodiment, the formed article essentially consists of or is made of the thermoplastic polymer material as described above. For example, the formed article is made from the thermoplastic polymer material via a thermal molding process, preferably via injection molding, blow molding or thermoforming, more preferably injection molding. It is possible to effectively sterilize an injection molded article having a complex shape and/or including openings and/or cavities.

Furthermore, it is possible that the formed article comprises, besides the thermoplastic polymer material, at least one other UV-C transparent polymer material, for example selected from polyolefins, such as polyethylene, polypropylene, or silicone. Further, it is possible that the formed article is combined (e.g. linked via screwing or adhesion) with another part made of UV-C transparent polymers, e.g. polyolefins or silicones. Advantageously, such combination can be used in the inventive process for sterilization, i.e. be sterilized in one step.

Further, it is possible that the formed article comprises one or more parts or sections of any arbitrary non UV-C transparent material, as long as said non UV-C transparent parts do not prevent inner surfaces from being sterilized. For example, such non UV-C transparent part may be located at one site of the formed article, that is opposite to the part made of the thermoplastic polymer material and opposite to the UV-C radiation source.

In a particularly preferred embodiment, the formed article is a medical device, preferably a disposable medical device. Preferably, the medical device can be sterilized by the inventive method directly after its production. For example, the formed article is a medical device selected from medical diagnostic devices, intravenous and catheter accessory, blood handling devices, chest drainage units, respiratory ventilating devices, medical filter housings, permanent device housings, tubes, connectors, fittings, and cuvettes. Said devices include but are not limited to luer locks, Y-sites, spikes, fittings, nozzles, protection caps and covers, blood plasma separators, collection and specimen vessels, and adapters, catheter accessories, chest drainage units, valve assemblies, meter housings, flow controls, filter housings, drip chambers, intravenous adapters, yankauers, rigid tubes, diagnostic cuvettes, diagnostic test packs, diagnostic rotors, optical sensor viewports, microfluidic devices, bracheotherapy needle hubs, inhalation mouthpieces and spacers.

Furthermore, it is possible to use the inventive method in any other area where formed articles made of the thermoplastic polymer material as described above are applicable and where disinfection or sterilization is required or desired, e.g. in production of food and dairy products, pharmaceutic or cosmetic products, or as well as for sanitary and bath equipment.

In another preferred embodiment, the formed article is selected from containers and/or packages used for cosmetic or pharmaceutical products or used in food industry, e.g. in production and packaging of beverages, meat products, or dairy products.

The formed article according to the invention, for example for medical use, can be prepared from the thermoplastic polymer material via commonly known processes, in particular thermal molding processes or by processing via the elastoviscous state, for example kneading, rolling, calendering, extrusion, injection molding, blow molding or thermoforming, in particular injection molding, being particularly preferred here.

The injection molding or extrusion of the thermoplastic polymer material can be effected in a manner known per se at temperatures in a range adapted to the thermoplastic polymer material.

In this context, the present invention is preferably directed the formed article, sterilized in the inventive method for UV sterilization, wherein the formed article prepared from the thermoplastic polymer material via extrusion, injection molding, blow molding and/or thermoforming, of the thermoplastic polymer material.

### Use of thermoplastic polymer material and formed article in UV sterilization

Furthermore, the present invention is directed to the use of an thermoplastic polymer material, which comprises at least 50 wt.-%, preferably at least 60 wt.-%, more preferably at least 70 wt.-%, based on the total polymer material, of at least one thermoplastic polymer A, selected from cyclic polyolefins, such as cyclic olefin polymers (COP), cyclic olefin copolymers COC and cyclic block copolymers CBC; aliphatic polyamides, aliphatic polyurethanes and halogenated polyolefins, for the production of formed articles, which are subject to a UV sterilization process, wherein the thermoplastic polymer material has a transmittance of at least 10 %, preferably at least 15 %, more preferably at least 20 %, also preferably at least 25 %, averaged over the wavelength range from 260 nm to 300 nm, and measured in accordance with ISO 13468-2 at a thickness of 3 mm. Preferably, the UV sterilization process includes the exposure of the outer surface of the formed article with UV radiation at a wavelength in the range of 260 nm to 300 nm.

Finally, a further aspect of the present invention is directed to the use of the inventive formed article as described above in a method for UV sterilization, wherein UV radiation at a wavelength in the range of 260 nm to 300 nm is utilized.

The description and preferred embodiments of the inventive method for UV sterilization and of the inventive formed article apply to the inventive use accordingly.

## Claims

1. Method for UV sterilization of a surface of a formed article, including sterilization of the inner surface of the formed article, comprising the steps:
a. providing a formed article, wherein at least one part of said formed article is made from a thermoplastic polymer material, which comprises at least 50 wt.-%, based on the thermoplastic polymer material, of at least one thermoplastic polymer A, selected from cyclic polyolefins; aliphatic polyamides, aliphatic polyurethanes and halogenated polyolefins;
b. exposure of the outer surface of the formed article with UV radiation at a wavelength in the range of 260 nm to 300 nm;
wherein the thermoplastic polymer material has a transmittance of at least 10 %, averaged over the wavelength range from 260 nm to 300 nm, and measured in accordance with ISO 13468-2 at a thickness of 3 mm.

2. Method according to Claim 1, wherein the thermoplastic polymer material has a transmittance of at least 8 %, preferably of at least 10 %, also preferably at least 15 %, more preferably at least 20 %, at a wavelength of 265 nm, measured in accordance with ISO 13468-2 at a thickness of 3 mm.

3. Method according to Claim 1 or 2, wherein the thermoplastic polymer material comprises from 50.0 to 100.0 wt.-%, preferably 60.0 to 99.9 wt.-%, more preferably 70.0 to 95.0 wt.-%, based on the total polymer material, of at least one thermoplastic polymer A selected from cyclic olefin polymer COP, cyclic olefin copolymer COC, cyclic block copolymer CBC; aliphatic polyamides, aliphatic polyurethanes and halogenated polyolefins.

4. Method according to any of Claims 1 to 3, wherein the thermoplastic polymer material comprises, based on the total weight of the thermoplastic polymer material:
A. 50.0 to 100.0 wt.-%, preferably 54.0 to 93.0 wt.-%, of at least one thermoplastic polymer A, selected from cyclic olefin polymer COP, cyclic olefin copolymer COC and cyclic block copolymer CBC; aliphatic polyamides, aliphatic polyurethanes and halogenated polyolefins,
B. 0.0 to 50.0 wt.-%, preferably 1.0 to 40.0 wt.-%, of at least one polymeric component B, different from A,
C. 0.0 to 20.0 wt.-%, preferably 1.0 to 10.0 wt.-%, of at least one plasticizer C, and
D. 0.0 to 10.0 wt.-%, preferably 0.0 to 5.0 wt.-%, of at least one further component D, preferably selected from additives, auxiliaries and/or fillers.

5. Method according to any of Claims 1 to 4, wherein the formed article is selected from medical devices; containers and/or packages used for cosmetic or pharmaceutical products or used in food industry.

6. Method according to any of Claims 1 to 5, wherein the UV radiation at a wavelength in the range of 260 to 300 nm is provided by one or more appropriate UV radiation sources, selected from UV light emitting diodes (UV-LEDs), wherein at least a significant amount of emission spectrum of the UV source, is in the range of 260 nm to 300 nm, preferably 260 to 280 nm.

7. Method according to any of Claims 1 to 6, wherein the exposure of the outer surface of the formed article with UV radiation is carried out in such way that a UV dose in the range of 1 to 10,000 mJ/cm² is effected.

8. Method according to any of Claims 1 to 7, wherein the method for UV sterilization inhibit the growth of one or more microorganisms of genus selected from *Escherichia, Salmonella, Listeria, Aspergillus, Bacillus, Cryptosporidium, Clostridium, Streptomyces, Aeromonas, Candida, Helicobacter, Klebsiella, Legionella, Listeria, Pseudomonas, Staphylococcus, Streptococcus, Lactobacillus, Bifidobacterium, Oenococcus,* and *Saccharomycodes.*

9. Method according to any of Claims 1 to 8, wherein the formed article is surrounded by a protective packaging during the UV exposure step b, wherein the protective packaging is made of an UV-transmissive polymer material.

10. Formed article sterilized in the method for UV sterilization according to any of claims 1 to 9, wherein at least one part of said formed article is made from an thermoplastic polymer material, which comprises at least 50 wt.-%, based on the thermoplastic polymer material, of at least one thermoplastic polymer A, selected from cyclic polyolefins, such as cyclic olefin polymer COP, cyclic olefin copolymer COC and cyclic block copolymer CBC; aliphatic polyamides, aliphatic polyurethanes and halogenated polyolefins; and wherein the thermoplastic polymer material has a transmittance of at least 10 % averaged over the wavelength range from 260 nm to 300 nm, and measured in accordance with ISO 13468-2 at a thickness of 3 mm.

11. Formed article according to Claim 10, wherein the formed article is selected from medical devices; containers and/or packages used for cosmetic or pharmaceutical products or used in food industry.

12. Formed article according to Claim 10 or 11, wherein the formed article is a medical device selected from medical diagnostic devices, intravenous and catheter accessory, blood handling devices, chest drainage units, respiratory ventilating devices, medical filter housings, permanent device housings, tubes, connectors, fittings, and cuvettes.

13. Formed article according to any of Claims 10 to 12, wherein the formed article is prepared from the thermoplastic polymer material via extrusion, injection molding, blow molding and/or thermoforming.
